## Europäisches Patentamt
## European Patent Office
## Office européen des brevets

(19) ⁂

(11) Numéro de publication: **0 005 655 B1**

# FASCICULE DE BREVET EUROPEEN

(45) Date de publication du fascicule de brevet:
**01.04.81**

(51) Int. Cl.³: **C 07 C 19/02,** C 07 C 17/15,
C 07 C 17/38, B 01 J 23/40,
B 01 D 53/36

(21) Numéro de dépôt: **79400210.5**

(22) Date de dépôt: **02.04.79**

---

(54) **Procédé d'oxyhydrochloration non polluant.**

---

(30) Priorité: **26.04.78 FR 7812256**

(43) Date de publication de la demande:
**28.11.79 Bulletin 79/24**

(45) Mention de la délivrance du brevet:
**01.04.81 Bulletin 81/13**

(84) Etats contractants désignés:
**BE DE FR GB IT NL**

(56) Documents cités:
**FR-A-2 217 063
FR-A-2 279 704
US-A-2 925 395
US-A-2 959 536
US-A-4 049 578**

(73) Titulaire: **RHONE-POULENC INDUSTRIES, 22, avenue
Montaigne, F-75008 Paris (FR)**

(72) Inventeur: **Brunelle, Jean-Pierre, Résidence Charcot
Zola 114, rue Emile Zola, F-94260 Fresnes (FR)**

(74) Mandataire: **Savina, Jacques et al, RHONE POULENC
Service Brevets Chimie et Polymères B.P. 753,
F-75360 Paris Cedex 08 (FR)**

---

Procédé d'oxyhydrochloration non polluant

La présente invention concerne un procédé d'oxyhydrochloration non polluant. Elle a plus particulièrement trait à un procédé d'oxyhdrochloration pour lequel les effluents gazeux résiduaires sont épurés par oxydation catalytique et lavage avant rejet dans l'atmosphère.

Les procédés classiques d'obtention des dérivés chlorés de l'éthylène et notamment du dichloro 1-2 éthane par oxyhydrochloration ne présentent pas des sélectivités égales à 100%; ainsi, l'effluent sortant du réacteur d'oxyhydrochloration contient toujours des quantités plus ou moins importantes de sous-produits. Ces sous-produits sont des mélanges complexes qui contiennent notamment, en plus de l'éthylène et de l'acide chlorhydrique n'ayant pas réagi, de l'oxyde de carbone, du gaz carbonique et des composés organiques chlorés tels que le chlorure d'éthyle, le dichloro 1-1 éthane, les trichloroéthanes, les tétrachloroéthanes, les dichloroéthylènes, le trichloroéthylène, le chloroforme, le tétrachlorure de carbone, le chloral, le perchloréthylène, etc.

La séparation des dérivés chlorés de l'éthylène de l'effluent sortant du réacteur d'oxyhydrochloration n'étant jamais, dans la pratique, quantitative, l'effluent résiduaire qui en résulte se trouve constitué d'azote, d'oxygène, ainsi que de quantités variables des dérivés chlorés de l'éthylène recherchés tels que le dichloro 1-2 éthane et de quantités variables des sous-produits précédemment nommés.

Pour traiter de tels effluents, il a déjà été proposé selon la demande de brevet français n° 2 279 704, d'injecter celui-ci dans un lit de catalyseur de combustion constitué par 0,01% à 0,5% en poids de platine ou de palladium et par 99,99 à 99,50% en poids d'un matériau choisi parmi Al$_2$O$_3$, SiO$_2$ ou un mélange d'Al$_2$O$_3$ et de SiO$_2$. Lorsque le métal utilisé est le palladium, la sélectivité en HCI et les rendements de conversion en CO et en dichloro 1-2 éthane obtenus selon ce procédé sont insuffisants, il se forme en plus de l'HCI du chlorure de vinyle ce qui est très gênant; par ailleurs, lorque le support est choisi parmi la silice, la silice alumine et divers types d'alumine comme l'alumine alpha, l'alumine activée, l'alumine gamma et la boehmite, les rendements de conversion de l'oxyde de carbone et/ou du dichloro 1-2 éthane sont également insuffisants. Selon la brevet américain n° 4 049 578, on connaît des catalyseurs de reforming comportant du platine et/ou de l'iridium déposés sur un support d'alumine êta. Selon les brevets américains n° 2 959 536 et 2 925 395 on connaît des catalyseurs de reforming, d'isomérisation, d'hydrogénation, d'hydrocracking, de déshydrogénation, d'oxydation du SO$_2$ etc. contenant du platine déposé sur un support d'alumine qui est converti en alumine êta lors de la calcination. Par ailleurs, il a déjà été proposé selon la demande de brevet français n° 2 279 703, de les faire passer à une température comprise entre 300 et 450°C sur un lit catalytique constitué d'un support d'alumine ou de silice sur lequel on a déposé 10 à 50% en poids d'oxyde de chrome. Cependant, un tel procédé présente notamment l'inconvénient de ne pas être sélectif et, en particulier, de favoriser la réaction de Deacon de formation du chlore moléculaire à partir d'acide chlorhydrique et d'oxygène. Cette formation de chlore moléculaire est, à la différence de l'acide chlorhydrique, extrêmement gênante car difficilement éliminable des effluents gazeux par les techniques classiques telles que le lavage à l'eau. De plus, la présence de chlore moléculaire en présence de vapeur d'eau peut conduire à des problèmes de corrosion des matériaux particulièrement gênants aux températures élevées. Enfin, les catalyseurs à base d'oxyde de chrome ne sont que d'une efficacité médiocre pour l'oxydation de l'oxyde carbone en gaz carbonique.

La demanderesse a mis au point un procédé permettant d'obvier à ces inconvénients et qui met en œuvre un catalyseur actif, stable et très sélectif pour l'oxydation de l'oxyde de carbone et des hydrocarbures chlorés ou non.

La présente invention concerne un procédé d'oxyhydrochloration non polluant dans lequel on met en réaction du gaz chlorhydrique, de l'air ou de l'oxygène, de l'éthylène et/ou un dérivé chloré de l'éthylène pour obtenir un effluent contenant des dérivés chlorés d'éthylène et caractérisé en ce que:

a) on sépare de l'effluent les dérivés chlorés de l'éthylène formés au cours de la réaction d'oxyhydrochloration, l'effluent résiduaire contenant essentiellement de l'oxygène, de l'azote, de l'oxyde de carbone, du gaz carbonique, de l'éthylène n'ayant pas réagi, du dichloro 1-2 éthane et au moins un hydrocarbure chloré pris dans le groupe constitué par: le chloroforme, le tétrachlorure de carbone, le chlorure d'éthyle, le dichloro 1-1 éthane, les trichloro 1-1-1 et 1-1-2 éthane, les tétrachloro 1-1-2-2 et 1-1-1-2 éthane, le chlorure de vinyle, les dichloro 1-1 et 1-2 éthylène, le trichloroéthylène, le perchloréthylène et le chloral;

b) on ajoute éventuellement un gaz diluant à l'effluent résiduaire;

c) on préchauffe l'effluent résiduaire à une température comprise entre 250 et 400°C;

d) on envoie cet effluent dans une zone de réaction adiabatique contenant un catalyseur d'oxydation constitué par du platine et/ou de l'iridium déposé(s) sur un support d'alumine de structure cristallographique êta, ce qui conduit après oxydation à un mélange de gaz renfermant essentiellement du gaz carbonique, de l'acide chlorhydrique, de la vapeur d'eau, de l'oxygène et de l'azote;

e) on utilise tout ou partie des calories dégagées lors de l'oxydation pour préchauffer, selon l'étape c), à l'aide d'un échangeur thermique, l'effluent issu de l'étape a);

f) on élimine par lavage l'acide chlorhydrique formé au cours de l'étape d'oxydation avant le rejet de l'effluent dans l'atmosphère ou son utilisation comme source de gaz inerte.

Le procédé d'oxyhydrochloration selon l'invention consiste à mettre en réaction du gaz chlorhydrique, de l'air ou de l'oxygène, de l'éthylène et/ou un dérivé chloré de l'éthylène pour former des dérivés chlorés de l'éthylène contenus dans un effluent.

La séparation de l'effluent des dérivés chlorés de l'éthylène formés au cours de la réaction d'oxyhydrochloration se compose généralement des étapes suivantes: lavage à l'eau de l'effluent pour en éliminer l'acide chlorhydrique non réagi, condensation par refroidissement des dérivés chlorés de l'éthylène et lavage de l'effluent par un solvant organique tel que l'hexachlorobutadiène.

Dans la pratique, ces différents traitements ne permettent pas de séparer quantitativement les dérivés chlorés de l'effluent gazeux. C'est ainsi que l'effluent résiduaire qui résulte de cette étape de séparation contient en plus de l'oxygène, de l'azote et de l'éthylène n'ayant pas réagi, des dérivés chlorés de l'éthylène non séparés tels que le dichloro 1-2 éthane ainsi que des quantités variables de différents sous-produits tels que l'oxyde de carbone, le gaz carbonique, le chloroforme, le tétrachlorure de carbone, le chlorure d'éthyle, le dichloro 1-1 éthane, les trichloro 1-1-1 et 1-1-2 éthane, le chlorure de vinyle, les dichloro 1-1 et 1-2 éthylène, le trichloroéthylène, le perchloroéthylène, le chloral, etc.

Si la séparation des dérivés chlorés de l'éthylène de l'effluent comprend une étape d'adsorption par un solvant organique tel que l'hexachlorobutadiène, des traces de ce solvant peuvent être présentes également dans l'effluent résiduaire à traiter. Généralement, les teneurs volumiques en éthylène, gaz carbonique, oxyde de carbone et produits organiques chlorés de l'effluent résiduaire à traiter sont respectivement de l'ordre de 0,5%–2%–1% et 0,5%, le produit organique chloré le plus abondant étant généralement le dichloro 1-2 éthane. Ces teneurs peuvent cependant varier dans de larges limites suivant les conditions de mise en œuvre. La teneur en oxygène de l'effluent résiduaire est généralement suffisante pour permettre dans une étape ultérieure l'oxydation des produits contenus dans celui-ci, c'est-à-dire l'oxyde de carbone, l'éthylène et les produits organiques chlorés. Dans le cas contraire, il est nécessaire selon le procédé de l'invention d'ajouter une quantité suffisante d'air ou d'oxygène à l'effluent résiduaire pour permettre l'oxydation totale des produits précédemment cités.

On ajoute éventuellement à l'effluent résiduaire un gaz diluant tel que l'azote ou l'air pour limiter l'élévation de température dans la zone de réaction adiabatique de l'étape subséquente. La quantité de gaz diluant mise en œuvre doit être telle que la température maximale dans la zone de réaction ne dépasse pas environ 600 °C et, de préférence, 500 °C. En effet, au-dessus de ces températures, la sélectivité des catalyseurs selon le procédé de la présente invention diminue du fait de la formation de chlore moléculaire selon la réaction de Deacon définie précédemment.

L'effluent résiduaire est préchauffé à une température comprise entre 250 et 400 °C puis est envoyé dans une zone de réaction adiabatique contenant un catalyseur d'oxydation constitué par du platine et/ou de l'iridium déposé(s) sur un support particulier d'alumine de structure cristallographique êta.

La vitesse spatiale de l'effluent définie par le rapport du débit volumique de l'effluent sur le volume de catalyseur est comprise entre 1000 et 100 000 heures$^{-1}$ et, de préférence, entre 5000 et 20 000 heures$^{-1}$.

On peut mettre en œuvre la réaction d'oxydation à la pression atmosphérique. Toutefois, il peut être, dans certains cas, avantageux d'opérer à une pression supérieure atmosphérique, et, en particulier, à une pression comprise entre 1 et 10 bars.

Le support utilisé pour la préparation des catalyseurs selon le procédé de l'invention est constitué d'alumine dont la structure cristallographique correspond essentiellement à la forme êta et dont la surface spécifique est comprise entre 200 et 400 m$^2$/g. Ce type d'alumine est connu de l'homme de l'Art. On pourra, par exemple, trouver quelques principes de sa préparation dans la revue technique de la Société Alcoa sur les oxydes et hydroxydes d'aluminium «Technical paper n° 19, K. Wefers et G.M. Bell, 1972, p. 14, paragraphe 2.22, p. 40, paragraphe 4.12». A titre d'illustration, le mode opératoire décrit ci-dessous peut être utilisé selon l'invention.

On prépare un gâteau de gel d'alumine à 20% d'alumine comptée en $Al_2O_3$ par essorage, lavage et filtration d'une suspension d'hydroxyde d'aluminium obtenue par précipitation en continu d'une solution d'aluminate de sodium à 100 g/l d'alumine comptée en $Al_2O_3$ par une solution d'acide nitrique de concentration telle et en quantité telle que ladite suspension titre environ 50 g/l d'alumine comptée en $Al_2O_3$ et que le rapport molaire

$$\frac{NO_3}{Al_2O_3}$$

soit égal à 0,20; le pH de précipitation se situe alors approximativement aux environs de 8,5. Ce gâteau est ensuite remis en suspension dans une solution ammoniacale 2 N de pH environ égal à 10 de manière à obtenir une suspension épaisse de gel d'alumine titrant 10 à 15% d'alumine. La pâte est maintenue sous agitation pendant environ 20 heures à 50 °C, ce qui permet d'obtenir une évolution de plus de 50% de l'hydroxyde d'aluminium en bayérite. Après séchage, mise en forme et calcination en atmosphère sèche vers 450 °C on obtient un support d'alumine êta dont la surface spécifique est de l'ordre de 350 m$^2$/g et dont l'acidité superficielle est très supérieue à celle des autres formes cristallographiques de l'alumine:

chi, gamma, delta, thêta, alpha...

Les catalyseurs d'oxydation qui sont mis en œuvre selon le procédé de l'invention contiennent de 0,02 à 5% en poids de platine et/ou d'iridium rapporté au support. Une teneur comprise entre 0,1 et 0,5% en poids de platine et/ou d'iridium est toutefois préférée. Lorsque l'on met en œuvre du platine et de l'iridium, la proportion relative en poids de platine par rapport à l'iridium pourra varier dans de larges limites et sera comprise de préférence entre $\frac{1}{10}$ et $\frac{10}{1}$.

Le catalyseur d'oxydation selon le procédé de l'invention peut être préparé selon les méthodes courantes, et, en particulier, par imprégnation du support au moyen de solutions de précurseurs minéraux ou organiques du platine et/ou de l'iridium. L'imprégnation peut être faite en une ou plusieurs étapes au moyen de solutions contenant des composés métalliques, du platine et/ou de l'iridium.

Comme composés solubles du platine et/ou de l'iridium classiquement utilisés, on peut citer: l'acide chloroplatinique, l'acide chloroïridique, le chlorure de platine tétramine, etc.

Après imprégnation du support avec la solution précédemment décrite, le catalyseur est ensuite séché, puis calciné dans un courant d'air à une température comprise entre 200 et 800°C pendant quelques heures. Le catalyseur peut être avantageusement traité dans une atmosphère contenant un dérivé soufré tel que l'hydrogène sulfuré. Dans ce cas, l'opération de réduction ou de sulfuration pourra se faire à l'une quelconque des étapes de préparation du catalyseur, après l'imprégnation du support, mais de préférence avant l'étape de calcination.

Le catalyseur pourra être mis en œuvre sous forme de sphères, d'extrudés, de pastilles ou sous toute autre forme.

Les réactions d'oxydation étant exothermique, il se produit, dans la zone réactionnelle, un dégagement de calories qui se traduit par une élévation de la température de l'effluent. On utilise tout ou partie des calories ainsi dégagées pour préchauffer, au moyen d'un échangeur thermique l'effluent résiduaire à une température comprise entre 250 et 400°C.

Le procédé selon la présente invention permet ainsi d'oxyder l'oxyde de carbone et les hydrocarbures chlorés ou non contenus dans l'effluent résiduaire et de conduire à un gaz exempt de chlore moléculaire et constitué essentiellement de vapeur d'eau, de gaz carbonique, d'acide chlorhydrique, d'oxygène et d'azote. L'effluent en résultant est ensuite traité, avant son rejet dans l'atmosphère ou son utilisation comme source de gaz inerte, pour en éliminer l'acide chlorhydrique. Cette élimination est réalisée habituellement dans une tour de lavage par absorption de l'acide chlorhydrique par une solution aqueuse de pH neutre ou alcalin.

Tout ou partie de l'effluent lavé peut être avantageusement recyclé à l'étape a) et jouer le rôle de diluant de l'effluent résiduaire avant l'étape d'oxydation.

Le catalyseur d'oxydation sélective des hydrocarbures chlorés selon le procédé de l'invention peut être utilisé tout aussi efficacement dans d'autres domaines que la fabrication de dérivés chlorés de l'éthylène par oxyhydrochloration et, en particulier, dans des domaines tels que le nettoyage à sec, le dégraissage des métaux, la teinture et, plus généralement, dans tout domaine mettant en œuvre des produits organiques chlorés contenant de 1 à 10 atomes de carbone.

Les exemples suivants, donnés à titre non limitatif, illustrent l'invention et, en particulier, l'intérêt des catalyseurs mis en œuvre selon le procédé de l'invention par rapport au catalyseur de l'art antérieur ainsi que la spécificité de la nature du support et des métaux utilisés.

Exemple 1:

Cet exemple donné à titre comparatif illustre l'intérêt du procédé selon l'invention par rapport au procédé de l'art antérieur mettant en œuvre un catalyseur à base d'oxyde de chrome déposé sur un support d'alumine.

Nous décrivons ci-dessous la préparation de deux catalyseurs selon le procédé de l'invention: 100 g d'extrudés d'alumine de structure êta, présentant une surface spécifique de 300 m²/g et un volume poreux de 0,55 cm³/g sont imprégnés par 55 cm³ d'une solution aqueuse d'acide chloroplatinique contenant 0,20 g de platine ou d'acide chloroïridique contenant 0,20 g d'iridium. Après quelques heures de contact, les extrudés sont séchés à 120°C puis calcinés sous air à 500°C pendant 3 heures.

Les catalyseurs A et B obtenus contiennent respectivement 0,2% en poids de platine ou 0,2% en poids d'iridium déposé sur un support d'alumine êta.

Nous décrivons maintenant la préparation d'un catalyseur C à base d'oxyde de chrome et revendiqué dans l'art antérieur: 100 g d'extrudés d'alumine êta sont imprégnés par 55 cm³ d'une solution aqueuse d'anhydride chromique contenant 20 g d'oxyde de chrome comptés en $Cr_2O_3$. Après quelques heures de contact, les extrudés sont séchés à 120°C puis calcinés sous air pendant 3 heures. Le catalyseur obtenu contient 20% en poids d'oxyde de chrome déposé sur un support d'alumine êta.

L'activité et la sélectivité des 3 catalyseurs précédents sont mesurées à une vitesse spatiale de 5000 heures$^{-1}$, sur un gaz synthétique représentatif des effluents résiduaires d'oxyhydrochloration et constitué de 1% d'oxyde de carbone, de 0,5% en volume de dichloro 1-2 éthane, de 5% en volume d'oxygène et 93,5% d'azote. Les conversions de l'oxyde de carbone mesurées à 300°C et du dichloro 1-2 éthane mesurées à 400°C ont été rassemblées dans le Tableau I. Ont été également reportées sur ce tableau, la sélectivité des trois catalyseurs pour la réaction d'oxydation du dichloroéthane en acide chlorhydrique, gaz carbonique et vapeur d'eau ainsi éventuellement que les autres produits chlorés formés.

0 005 655

Tableau I

|  | | | % Conversion du | | % Sélectivité en HCl | Produit formé autre que HCl |
|---|---|---|---|---|---|---|
|  | | | CO à 300 °C | dichloro 1–2 éthane à 400 °C | à 400 °C | |
| Invention | Catalyseurs | A | 80 | 95 | 100 | Néant |
|  | | B | 90 | 95 | 100 | Néant |
| Art Antérieur | | C | 0 | 75 | 85 | Chlore Moléculaire |

L'examen de ces résultats montre très nettement la supériorité des deux catalyseurs A et B selon le procédé de l'invention sur le catalyseur C de l'art antérieur à base d'oxyde de chrome et, plus particulièrement, en ce qui concerne leur activité oxyc ite vis à vis de l'oxyde de carbone. De plus, la sélectivité des deux catalyseurs A et B selon le procédé de l'invention est égale à 100% alors que celle du catalyseur C de l'art antérieur n'est que de 85%, 15% du dichloroéthane étant dans ce cas transformé en chlore moléculaire selon la réaction de Deacon.

Un test de 128 heures effectué dans les conditions précédemment décrites montre, par ailleurs, que les performances catalytiques des catalyseurs A et B selon le procédé de l'invention sont parfaitement stables.

Exemple 2:

Dans cet exemple, nous décrivons la préparation de plusieurs catalyseurs à base de platine déposés sur différents supports afin de montrer la très grande supériorité et spécificité du support revendiqué pour la préparation des catalyseurs selon le procédé de l'invention, c'est-à-dire du support d'alumine de structure cristallographique êta.

Le mode opératoire décrit dans l'exemple 1, utilisé pour préparer le catalyseur A de l'invention est reproduit en utilisant les supports suivants:

D – des billes d'alumine de structure alpha dont la surface spécifique est égale à 10 m²/g et le volume poreux à 0,50 cm³/g;

E – des billes d'alumine activée obtenues selon le procédé décrit dans le brevet US n° 2 915 365 et présentant une surface spécifique de 250 m²/g et un volume poreux de 0,50 cm³/g;

F – des extrudés de silice-alumine (support présentant une importante acidité de surface) contenant 70% de silice et présentant une surface spécifique de 350 m²/g et un volume poreux de 0,80 cm³/g;

G – des pastilles de silice de 400 m²/g dont le volume poreux est égal à 0,60 cm³/g;

H – des billes d'alumine de structure gamma dont la surface spécifique est égale à 200 m²/g et le volume poreux égal à 0,50 cm³/g;

I – des billes de monohydrate d'alumine de structure boehmitique dont la surface spécifique est égale à 50 m²/g et le volume poreux à 0,50 cm³/g.

Après imprégnation par un volume égal au volume poreux du support d'une solution aqueuse d'acide chloroplatinique contenant 0,20 g de platine, les catalyseurs sont séchés à 120 °C puis calcinés à 500 °C sous air pendant 3 heures: Les catalyseurs D, E, F, G, H et I respectivement obtenus contiennent tous 0,2% en poids de platine.

Les performances de ces catalyseurs mesurées dans les conditions décrites dans l'exemple 1 ont été reportées dans le Tableau II:

Tableau II

|  | | | Nature du Support | % Conversion | |
|---|---|---|---|---|---|
|  | | | | de l'oxyde de carbone à 300 °C | du dichloro 1–2 éthane à 400 °C |
| Invention | | A | Alumine êta | 80 | 95 |
|  | | B | Alumine êta | 90 | 95 |
| Comparatif | Catalyseurs | D | Alumine alpha | 85 | 0 |
|  | | E | Alumine activée | 35 | 55 |
|  | | F | Silice-Alumine | 20 | 30 |
|  | | G | Silice | 20 | 10 |
|  | | H | Alumine Gamma | 70 | 65 |
|  | | I | Boehmite | 65 | 35 |

Les résultats reportés dans le Tableau II indiquent clairement que la spécificité du support d'alumine de structure êta conduit à des catalyseurs à base de platine ou d'iridium nettement plus actifs, selon le procédé de l'invention, que ceux mettant en œuvre d'autres supports d'alumine, de silice ou de silice-alumine. Ceci est particulièrement net lorsque l'on compare les conversions du dichloro 1-2 éthane mesurées à 400°C.

Exemple 3:

Nous décrivons ci-dessous la préparation de différents catalyseurs se différenciant des catalyseurs selon le procédé de l'invention non plus par la nature du support mais par la nature de la phase métallique.

Le mode opératoire suivi dans l'Exemple 1 pour la préparation des catalyseurs A et B selon le procédé de l'invention est reproduit avec pour seule différence l'utilisation, non plus d'une solution d'acide chloroplatinique ou d'acide chloroïridique mais:
– d'une solution de chlorure de palladium contenant 0,20 g de palladium,
– d'une solution de trichlorure de ruthénium contenant 0,20 g de ruthénium,
– d'une solution de trichlorure de rhodium contenant 0,20 g de rhodium;
– d'une solution de nitrate de cuivre contenant 20 g d'oxyde cuivrique.

Les catalyseurs J, K, L, M obtenus selon ce mode opératoire contiennent respectivement 0,2% en poids de palladium, 0,2% en poids de ruthénium, 0,2% en poids de rhodium et 20% en poids d'oxyde cuivrique déposés sur le support d'alumine de structure êta décrit dans l'exemple 1.

Leur activité oxydante est ensuite mesurée à une vitesse spatiale de 5000 heures[-1]. Le gaz utilisé est comme dans l'exemple 1 constitué de 1% en volume d'oxyde de carbone, de 0,5% en volume de dichloro 1-2 éthane, de 5% d'oxygène et de 93,5% d'azote.

Les conversions du CO mesurées à 300°C et du dichloro 1-2 éthane mesurées à 400°C sont rassemblées dans le tableau III ainsi que les sélectivités des différents catalyseurs pour la réaction d'oxydation du dichloro 1-2 éthane en acide chlorhydrique, gaz carbonique et vapeur d'eau.

Lorsque la sélectivité des catalyseurs n'est pas égale à 100%, la nature des produits chlorés formés autres que l'acide chlorhydrique est indiquée (chlore moléculaire et chlorure de vinyle).

Tableau III

| | | Nature du Métal | % Conversion du | | % Sélectivité en HCl | Produit Chloré formé autre que HCl |
|---|---|---|---|---|---|---|
| | | | CO à 300°C | dichloro 1–2 éthane à 400°C | à 400°C | |
| Invention | Catalyseurs | A Pt | 80 | 95 | 100 | Néant |
| | | B Ir | 90 | 95 | 100 | Néant |
| Comparatif | | J Pd | 20 | 85 | 30 | Chlorure de vinyle |
| | | K Ru | 15 | 85 | 20 | Chlorure de vinyle |
| | | L Rh | 30 | 80 | 50 | chlorure de vinyle |
| | | M Cu | 5 | 20 | 95 | Chlore moléculaire |

L'examen des résultats reportés dans le Tableau III montre très nettement la spécificité des catalyseurs A et B selon le procédé de l'invention et contenant du platine ou de l'iridium par rapport aux catalyseurs mettant en œuvre d'autres métaux précieux tels que le ruthénium, le rhodium ou le palladium ou à des métaux de transition non nobles réputés actifs dans les réactions classiques d'oxydation tels que le cuivre.

La supériorité des catalyseurs A et B de l'invention sur les catalyseurs J, K, L et M est particulièrement évidente lorsque l'on compare leur activité mesurée à 300°C pour l'oxydation de l'oxyde de carbone ainsi que leur sélectivité mesurée à 400°C pour la réaction d'oxydation du dichloro 1-2 éthane.

**Revendications**

1. Procédé d'oxyhydrochloration non polluant du type selon lequel on met en réaction du gaz chlorhydrique, de l'air ou de l'oxygène, de l'éthylène et/ou un dérivé chloré de l'éthylène pour obtenir un effluent contenant des dérivés chlorés d'éthylène puis:

a) on sépare de l'effluent les dérivés chlorés de l'éthylène formés au cours de la réaction d'oxyhydrochloration, l'effluent résiduaire contenant essentiellement de l'oxygène, de l'azote, de l'oxyde de carbone, du gaz carbonique, de l'éthylène n'ayant pas réagi, du dichloro 1-2 éthane et au moins un hydrocarbure chloré pris dans le groupe constitué par: le chloroforme, le tétrachlorure de carbone, le chlorure d'éthyle, le dichloro 1-1 éthane, les trichloro 1-1-1 et 1-1-2 éthane, les tétrachloro 1-1-2-2 et 1-1-1-2 éthane, le chlorure de vinyle, les dichloro 1-1 et 1-2 éthylène, le trichloroéthylène, le perchloréthylène et le chloral;

b) on ajoute éventuellement un gaz diluant à l'effluent résiduaire;

c) on préchauffe l'effluent résiduaire à une température comprise entre 250 et 400°C;

d) on envoie cet effluent dans une zone de réaction adiabatique contenant un catalyseur, ce qui conduit à un mélange de gaz renfermant essentiellement du gaz carbonique, de l'acide chlorhydrique, de la vapeur d'eau, de l'oxygène et de l'azote;

e) on utilise tout ou partie des calories dégagées lors de l'oxydation pour préchauffer, selon l'étape c), à l'aide d'un échangeur thermique, l'effluent issu de l'étape a);

f) on élimine par lavage l'acide chlorhydrique formé au cours de l'étape d'oxydation avant le rejet de l'effluent dans l'atmosphère de son utilisation comme source de gaz inerte, caractérisé en ce que le catalyseur est constitué par du platine et/ou de l'iridium déposé(s) sur un support d'alumine de structure cristallographique êta.

2. Procédé selon la revendication 1, caractérisé en ce que la surface spécifique du support d'alumine de structure cristallographique êta est comprise entre 200 et 400 m²/g.

3. Procédé selon la revendication 1, caractérisé en ce que le catalyseur contient de 0,02% à 5% en poids de platine et/ou d'iridium rapporté(s) au support.

4. Application d'un catalyseur constitué de platine et/ou d'iridium déposé sur un support d'alumine de structure cristallographique êta à l'oxydation sélective des hydrocarbures chlorés.

**Patentansprüche**

1. Verfahren zur umweltfreundlichen Oxyhydrochlorierung, bei der man Chlorwasserstoffgas, Luft oder Sauerstoff, Äthylen und/oder ein Chlorderivat des Äthylens miteinander reagieren lässt, um einen austretenden Produktstrom zu erhalten, der Chlorderivate des Äthylens enthält und

a) aus dem Produktstrom die bei der Oxyhydrochlorierung gebildeten Chlorderivate des Äthylens abtrennt, wobei das Restgas im wesentlichen Sauerstoff, Stickstoff, Kohlenoxid, Kohlendioxid, nicht reagiertes Äthylen, Dichlor-1-2-äthan und wenigstens einen chlorierten Kohlenwasserstoff der folgenden Gruppe enthält: Chloroform, Tetrachlorkohlenstoff, Äthylenchlorid, Dichlor-1-1-äthan, die Trichlor-1-1-1 und 1-1-2-äthane, die Tetrachlor-1-1-2-2 und 1-1-1-2-Äthane, Vinylchlorid, die Dichlor-1-1 und 1-2-Äthylene, Trichloräthylen, Perchloräthylen und Chloral;

b) dem Restgas eventuell ein Verdünnungsgas zusetzt;

c) das Restgas auf eine Temperatur zwischen 250 und 400°C erhitzt;

d) diesen Gasstrom in eine einen Katalysator enthaltende adiabatische Reaktionszone leitet, was zu einem Gasgemisch führt, das im wesentlichen Kohlendioxid, Salzsäure, Wasserdampf, Sauerstoff und Stickstoff enthält;

e) einen Teil oder die gesamten bei der Oxydation frei werdenden Kalorien verwendet, um gemäss der Stufe c) mit Hilfe eines Wärmeaustauschers, das aus der Stufe a) austretende Restgas vorzuheizen;

f) die bei der Oxydation gebildete Salzsäure durch Wäsche entfernt bevor der Gasstrom in die Atmosphäre geleitet oder als Inertgasquelle verwendet wird,
dadurch gekennzeichnet, dass der Katalysator aus Platin und/oder Iridium besteht, das auf einem Träger aus Aluminiumoxid der Kristallstruktur eta niedergeschlagen ist.

2. Verfahren nach Anspruch 1, dadurch gekennzeichnet, dass die spezifische Oberfläche des Aluminiumoxidträgers mit Etastruktur zwischen 200 und 400 m²/g liegt.

3. Verfahren nach Anspruch 1, dadurch gekennzeichnet, dass der Katalysator 0,02 bis 5 Gewichtsprozent Platin und/oder Iridium, bezogen auf den Träger, enthält.

4. Verwendung eines Katalysators der Platin und/oder Iridium auf einem Aluminiumoxidträger der Kristallstruktur eta enthält zur selektiven Oxydation von chlorierten Kohlenwasserstoffen.

**Claims**

1. Oxyhydrochlorination process which does not cause pollution, of the type in which hydrogen chloride, air or oxygen, and ethylene and/or a chlorinated derivative of ethylene are reacted in order to produce an effluent containing chlorinated derivatives of ethylene, and then:

a) the chlorinated derivatives of ethylene formed during the oxyhydrochlorination reaction are separated from the effluent, the residual effluent essentially containing oxygen, nitrogen, carbon monoxide, carbon dioxide, unreacted ethylene, 1,2-dichloroethane and at least one chlorohydrocarbon from the group comprising: chloroform, carbon tetrachloride, ethyl chloride, 1,1-dichloroethane, 1,1,1- and 1,1,2-trichloroethane, 1,1,2,2- and 1,1,1,2-tetrachloroethane, vinyl chloride, 1,1- and 1,2-dichloroethylene, trichloroethylene, perchloroethylene and chloral;

b) a diluent gas is optionally added to the residual effluent;

c) the residual effluent is preheated to a temperature between 250 and 400°C;

d) this effluent is sent into an adiabatic reaction zone containing a catalyst, this leading to a mixture of gases essentially containing carbon dioxide, hydrochloric acid, steam, oxygen and nitrogen;

e) all or part of the heat evolved during the oxidation is used to preheat, in accordance with step

c) and with the aid of a heat exchanger, the effluent produced from step a); and

f) the hydrochlorid acid formed during the oxidation step is removed by washing before the effluent is released into the atmosphere or used as a source of inert gas, characterised in that the catalyst consists of platinum and/or iridium deposited on a support of alumina having the eta crystallographic structure.

2. Process according to Claim 1, characterised in that the specific surface area of the support of alumina having the eta crystallographic structure is between 200 and 400 m²/g.

3. Process according to Claim 1, characterised in that the catalyst contains from 0.02% to 5% by weight of platinum and/or iridium, relative to the support.

4. Application of a catalyst consisting of platinum and/or iridium deposited on a support of alumina having the eta crystallographic structure, to the selective oxidation of chlorohydrocarbons.